# EUROPEAN PATENT APPLICATION

(11) **EP 4 703 729 A1**
(43) Date of publication of application: **04.03.2026**
(21) Application number: 24797200.3
(22) Date of filing: 26.04.2024
(51) Int. Cl.: G01N 33/68, G01N 33/53, G01N 33/564

(54) **METHOD FOR DETERMINING IGA NEPHROPATHY**

(30) Priority: 26.04.2023 JP 2023072454
(71) Applicant: Oriental Yeast Co., Ltd., Tokyo 174-8505 (JP); Fujita Academy, Toyoake-shi, Aichi 470-1192 (JP)
(72) Inventor: TAKAHASHI, Kazuo, Toyoake-shi, Aichi 470-1192 (JP); OHYAMA, Yukako, Toyoake-shi, Aichi 470-1192 (JP); ENOMOTO, Tetsuro, Nagahama-shi, Shiga 526-0804 (JP); YAMAMOTO, Keiji, Nagahama-shi, Shiga 526-0804 (JP)
(74) Representative: Vossius & Partner Patentanwälte Rechtsanwälte mbB
(86) International application number: PCT/JP2024/016523
(87) International publication number: WO 2024/225457

(57) **Abstract**

The present invention provides a method for determining a likelihood of onset or disease activity of IgA nephropathy by blood-mediated liquid biopsy, instead of highly invasive kidney biopsy, or conventionally used proteinuria or hematuria. The invention is a method for determining a likelihood of onset or disease activity of IgA nephropathy in a subjected animal, including (a) measuring an IgA nephropathy marker protein contained in an IgA immune complex in a blood sample collected from a subjected animal; and (b) determining the likelihood of onset or disease activity of IgA nephropathy in the subjected animal based on an amount of the IgA nephropathy marker protein measured in step (a), wherein the IgA nephropathy marker protein is a protein contained in an IgA immune complex, and a protein that is contained in a greater amount in an IgA immune complex of an IgA nephropathy patient group than in an IgA immune complex of a healthy subject group.

## Description

### TECHNICAL FIELD

The present invention relates to a method for determining a likelihood of onset or disease activity of IgA nephropathy by blood-mediated liquid biopsy.

The present application claims priority based on Japanese Patent Application No. 2023-072454, filed on April 26, 2023, the contents of which are incorporated herein by reference.

### BACKGROUND ART

Immunoglobulin A (IgA) nephropathy is a chronic glomerulonephritis characterized by deposition of IgAl and complement C3 in the glomerular mesangial region, mesangial cell proliferation, and matrix proliferation. Autoantibodies are formed against abnormal glycosylated IgA1, and immune complexes containing galactose-deficient IgA1 and its autoantibodies are formed in the blood. It is believed that deposition of the formed immune complexes in the mesangial region of the glomerulus induces nephritis and leads to renal damage. IgA nephropathy is the most common primary glomerulonephritis, but approximately 20-40% of cases progress to end-stage renal failure, requiring renal replacement therapy such as hemodialysis, 20-30 years after renal biopsy. Therefore, early diagnosis and treatment, as well as accurate assessment of disease activity, are essential for IgA nephropathy.

The initial symptom of IgA nephropathy is mainly abnormal urinalysis, and hematuria or proteinuria during a health check is often the trigger for discovery. A definitive diagnosis of IgA nephropathy requires a pathological diagnosis based on renal histological findings obtained through a renal biopsy, but renal biopsy requires hospitalization and is highly invasive. It is not easy to perform frequent renal biopsies on the same patient for the purpose of assessing disease activity. Meanwhile, while the traditional methods of assessing disease activity based on proteinuria, hematuria, or serum creatinine are simple and useful, they have limitations in terms of accurate diagnosis.

Currently, the treatment of IgA nephropathy mainly includes supportive treatment centered on RA (renin-angiotensin) system inhibitors, and aggressive treatment that combines supportive treatment with immunosuppressants such as corticosteroids or tonsillectomy. Because the aggressive treatment is highly invasive and carries the risk of complications, there is a need for a simple test, such as a serum test, that can diagnose IgA nephropathy or assess disease activity as information for determining whether to perform aggressive treatment at the time of initial onset and recurrence.

IgA nephropathy is known to involve two complement pathways: an alternative pathway and a lectin pathway. It has been reported that the serum concentration of complement factor H-related protein 1 (FHRP1), one of the regulatory factors in the blood of the alternative pathway, tends to be elevated in patients with IgA nephropathy compared to healthy individuals (Non-Patent Document 1). However, the difference in serum concentrations between healthy individuals and patients with IgA nephropathy is slight, making it difficult to use clinically as a marker for the onset of IgA nephropathy.

### Citation List

### Patent Document

Non-Patent Document 1: Medjeral-Thomas, et al., Kidney International, 2017, vol.92(4), p.942-952.

### SUMMARY OF INVENTION

### Technical Problem

The objective of the present invention is to provide a method for determining a likelihood of onset or disease activity of IgA nephropathy by blood-mediated liquid biopsy, instead of highly invasive renal biopsy, or conventionally used proteinuria or hematuria.

### Solution to Problem

As a result of intensive research aimed at solving the above-mentioned problems, the present inventors have succeeded in identifying a new protein that shows high levels specifically in IgA nephropathy patients by analyzing samples from IgA nephropathy patients and samples from people without IgA nephropathy using a mass spectrometer. The inventors have found that by using this protein as a marker, useful information can be obtained on the presence or absence of onset of IgA nephropathy and the state of the disease pathology, and have completed the invention.

That is, a method for determining IgA nephropathy and a kit for determining IgA nephropathy according to the present invention are as follows.
[1] A method for determining a likelihood of onset or disease activity of IgA nephropathy in a subjected animal, including:
   (a) measuring an IgA nephropathy marker protein contained in an IgA immune complex in a blood sample collected from a subjected animal; and
   (b) determining a likelihood of onset or disease activity of IgA nephropathy in the subjected animal based on an amount of the IgA nephropathy marker protein measured in step (a), wherein
      the IgA nephropathy marker protein is a protein contained in an IgA immune complex, and a protein that is contained in a greater amount in an IgA immune complex of an IgA nephropathy patient group than in an IgA immune complex of a healthy subject group.
[2] The method for determining IgA nephropathy according to [1], wherein
   step (a) is carried out by:
   (c-1) separating serum or plasma from the blood sample collected from the subjected animal;
   (c-2) recovering the IgA immune complex contained in the serum or the plasma obtained in step (c-1); and
   (c-3) measuring the IgA nephropathy marker protein in the IgA immune complex recovered in step (c-2).
[3] The method for determining IgA nephropathy according to [1], wherein
   step (a) is carried out by:
   (c-1) separating serum or plasma from the blood sample collected from the subjected animal;
   (c-2) recovering the IgA immune complex contained in the serum or the plasma obtained in step (c-1);
   (c-3) measuring the IgA nephropathy marker protein in the IgA immune complex recovered in step (c-2);
   (c-4) measuring an IgA protein in the IgA immune complex recovered in step (c-2);
   (c-5) dividing the amount of the IgA nephropathy marker protein measured in step (c-3) by an amount of the IgA protein measured in step (c-4), and determining the amount of the IgA nephropathy marker protein contained in the IgA immune complex in the blood sample collected from the subjected animal.
[4] The method for determining IgA nephropathy according to [2] or [3], wherein
   in step (c-2), the recovery of IgA immune complex is carried out by affinity chromatography using an anti-IgA antibody.
[5] The method for determining IgA nephropathy according to any one of [1] to [4], wherein
   the IgA nephropathy marker protein in the IgA immune complex is measured by an immunological technique using an antibody against the IgA nephropathy marker protein.
[6] The method for determining IgA nephropathy according to [5], wherein
   the immunological technique is ELISA, latex agglutination, or immunochromatography.
[7] The method for determining IgA nephropathy according to any of [1] to [4], wherein
   the IgA nephropathy marker protein in the IgA immune complex is measured by liquid chromatography-mass spectrometry.
[8] The method for determining IgA nephropathy according to any one of [1] to [7], wherein
   the IgA nephropathy marker protein is one or more selected from the group consisting of alpha 1-antitrypsin, apolipoprotein C-1, FHRP1, cathepsin G, SLC13A4, alpha 2-macroglobulin, myeloperoxidase, C1QB, RhoGAP42, and C1QC.
[9] The method for determining IgA nephropathy according to any one of [1] to [8], wherein
   in step (b), if the amount of the IgA nephropathy marker protein measured in step (a) is greater than a predetermined threshold value for distinguishing between an IgA nephropathy patient group and a healthy subject group, the subjected animal is determined to have a high likelihood of onset of IgA nephropathy.
[10] The method for determining IgA nephropathy according to [9], wherein
   if the subjected animal is determined to have a high likelihood of onset of IgA nephropathy, it is predicted that corticosteroid treatment will be effective for the subjected animal.
[11] The method for determining IgA nephropathy according to [9], wherein
   if the subjected animal is determined to have a high likelihood of onset of IgA nephropathy, it is predicted that treatment with a complement inhibitor targeting an alternative pathway or a lectin pathway will be effective for the subjected animal.
[12] The method for determining IgA nephropathy according to any one of [1] to [11], wherein
   the subjected animal is an animal suspected of developing nephropathy.
[13] The method for determining IgA nephropathy according to any one of [1] to [12], wherein
   the subjected animal is an animal diagnosed with hematuria or proteinuria.
[14] The method for determining IgA nephropathy according to any one of [1] to [10], wherein
   in step (b), if the amount of the IgA nephropathy marker protein measured in step (a) is lower than the amount of the IgA nephropathy marker protein contained in the IgA immune complex in the blood sample collected from the subjected animal before a time the blood sample provided in step (a) was collected, it is determined that the pathological condition of IgA nephropathy in the subjected animal has improved.
[15] A kit for determining a likelihood of onset or disease activity of IgA nephropathy in a subjected animal, including:
   an anti-IgA antibody; and
   an antibody against an IgA nephropathy marker protein, wherein
   the IgA nephropathy marker protein is a protein contained in an IgA immune complex, and a protein that is contained in a greater amount in an IgA immune complex of an IgA nephropathy patient group than in an IgA immune complex of a healthy subject group.
[16] The kit for determining IgA nephropathy according to [15], wherein
   the IgA nephropathy marker protein is one or more selected from the group consisting of alpha 1-antitrypsin, apolipoprotein C-1, FHRP1, cathepsin G, SLC13A4, alpha 2-macroglobulin, myeloperoxidase, C1QB, RhoGAP42, and C1QC.
[17] The kit for determining IgA nephropathy according to [15] or [16], used to determine the likelihood of onset of IgA nephropathy in a subjected animal.
[18] The kit for determining IgA nephropathy according to [15] or [16], wherein
   the subjected animal is an animal diagnosed with IgA nephropathy, and
   the kit is used to obtain information for selecting a treatment for the subjected animal.

### Advantageous Effects of Invention

The method for determining IgA nephropathy according to the present invention can provide useful information for assessing the presence or absence of IgA nephropathy or treatment methods using blood as a sample. This determination method does not require a kidney biopsy to be performed on the subjected animal, and is therefore expected to reduce the burden on patients and enable them to receive appropriate treatment.

By using the kit for determining IgA nephropathy according to the invention, the method for determining IgA nephropathy can be carried out more simply.

### BRIEF DESCRIPTION OF DRAWINGS

FIG. 1 shows the results of comparing the relative abundance of proteins in IgA immune complexes between an IgA nephropathy group (supportive treatment group + aggressive treatment group) (at the time of kidney biopsy) and a healthy subject group in Example 1.
FIG. 2 shows the results of comparing the proteins in IgA immune complexes before and after treatment in the aggressive treatment group for IgA nephropathy in Example 1.
FIG. 3 shows the results of measuring the amount of FHRP1 contained in IgA immune complexes (amount of FHRP1 contained per 10 µg of IgA [ng/10 µg of IgA]) in each group in Example 1.
FIG. 4 shows the results of comparing the amounts of FHRP1 (amount of FHRP1 contained per 10 µg of IgA [ng/IgA 10 µg]) before and after treatment in each group in Example 1.

### DESCRIPTION OF EMBODIMENTS

In the present invention and the present specification, the term "IgA nephropathy marker protein" refers to a protein contained in an IgA immune complex in blood, where the amount of the protein contained in an IgA immune complex of an IgA nephropathy patient group is greater than that in an IgA immune complex of a healthy subject group.

As described in the Examples below, the IgA nephropathy marker protein can be identified by the following method. First, blood samples are collected from an IgA nephropathy patient group who have been definitively diagnosed with IgA nephropathy by other means such as kidney biopsy, and from a healthy subject group, and the amounts of each protein contained in the IgA immune complexes in the blood samples are measured. The average amounts of proteins contained in the IgA immune complexes are calculated for the IgA nephropathy patients and the healthy subject group, and proteins whose average concentration in the IgA nephropathy patients is significantly higher than that in the healthy subject group can be identified as IgA nephropathy marker proteins.

In the invention and the specification, specific examples of the "IgA nephropathy marker protein" include alpha-1-antitrypsin, apolipoprotein C-1, FHRP1 (Complement factor H related protein 1), cathepsin G, SLC13A4 (Solute carrier family 13 member 4), alpha-2-macroglobulin, myeloperoxidase, C1QB (Complement C1q subcomponent subunit B), RhoGAP42 (Rho GTPase-activating protein 42), C1QC (Complement C1q subcomponent subunit C), and the like.

The method for determining IgA nephropathy according to the invention is a method for determining the likelihood of onset or disease activity of IgA nephropathy in a subjected animal, and includes the following steps:
(a) measuring an IgA nephropathy marker protein contained in an IgA immune complex in a blood sample collected from a subjected animal;
(b) determining the likelihood of onset or disease activity of IgA nephropathy in the subjected animal based on the amount of the IgA nephropathy marker protein measured in step (a).

The above steps (a) and (b) are steps carried out in vitro.

The IgA nephropathy marker protein used in the present invention may be one type, or two or more types may be used in combination. In the present invention, the IgA nephropathy marker protein may be one or more types selected from the group consisting of alpha1-antitrypsin, apolipoprotein C-1, FHRP1, cathepsin G, SLC13A4, alpha2-macroglobulin, myeloperoxidase, C1QB, RhoGAP42, and C1QC. In the present invention, it is particularly preferable to use FHRP1 as the IgA nephropathy marker protein.

In step (a), the IgA nephropathy marker protein contained in an IgA immune complex in a blood sample collected from a subjected animal is measured. In the present invention, by using the amount of the IgA nephropathy marker protein contained in an IgA immune complex in a blood sample as an index, rather than the amount of IgA nephropathy marker protein in the blood sample, it is possible to more accurately distinguish between IgA nephropathy patients and IgA nephropathy-free patients. Therefore, the method for determining IgA nephropathy according to the invention can accurately determine whether or not a subjected animal has developed IgA nephropathy. Furthermore, the method for determining IgA nephropathy according to the present invention can provide information useful for assessing disease activity in cases where the subject has IgA nephropathy.

The method for measuring the amount of the IgA nephropathy marker protein contained in the IgA immune complex in a blood sample collected from a subjected animal is not particularly limited. For example, serum or plasma can be prepared from the blood sample, the IgA immune complex contained therein can be recovered, and the IgA nephropathy marker protein in the recovered IgA immune complex can be measured.

Specifically, the measurement of the IgA nephropathy marker protein in the IgA immune complex in step (a) can be carried out, for example, by the following steps (c-1) to (c-3).
(c-1) separating serum or plasma from a blood sample collected from a subjected animal;
(c-2) recovering IgA immune complexes contained in the serum or plasma obtained in step (c-1);
(c-3) measuring the IgA nephropathy marker protein in the IgA immune complexes recovered in step (c-2).

**In** the method for determining IgA nephropathy according to the invention, the subjected animal is not particularly limited as long as it has kidneys. The subjected animal in the method for determining IgA nephropathy according to the present invention is preferably a mammal, more preferably a human, a laboratory animal such as mouse, rat, monkey, or the like, or a livestock or pet animal such as a rabbit, dog, cat, cow, horse, sheep, or the like, and particularly preferably a human.

The subjected animal in the method for determining IgA nephropathy according to the invention is preferably an animal suspected of developing nephropathy. Specific examples of the animals suspected of developing nephropathy include animals confirmed to have an increased amount of protein in urine or pathologies observed in nephropathy, such as hematuria, and particularly preferred are animals diagnosed with hematuria or proteinuria. For example, in the case of humans, if the amount of protein in urine is 0.15 g/day or more in terms of creatinine, the onset of nephropathy is suspected, and therefore such animals are suitable as subjected animals in the method for determining IgA nephropathy according to the invention.

Plasma or serum can be prepared from blood collected from a subjected animal by conventional methods. For example, blood can be collected from a subjected animal into a blood collection tube pre-containing an anticoagulant such as sodium citrate, heparin, EDTA, sodium fluoride, or the like, and then centrifuged to precipitate blood cell components and recover the supernatant plasma. Alternatively, the blood can be coagulated and then centrifuged to precipitate blood cell components and recover the supernatant serum. Blood collected from a subjected animal, or plasma or serum prepared therefrom, can be used for IgA immune complex recovery immediately after preparation, or can be stored for a predetermined period before use. Plasma or serum can be stored refrigerated or frozen, at room temperature, or lyophilized to form a frozen powder. Additionally, plasma or serum can be pretreated by diluting with an appropriate buffer or adding various additives. Examples of additives include surfactants, protease inhibitors, nuclease inhibitors, pH adjusters, pH indicators, and the like.

Recovery of IgA immune complexes contained in serum or plasma can be carried out by various methods as long as the concentration of IgA immune complexes is increased compared to before recovery. In particular, affinity chromatography using an anti-IgA antibody is preferable, as it allows recovery of IgA immune complexes with fewer impurities. The anti-IgA antibody is not particularly limited as long as it is capable of selectively binding to IgA in IgA immune complexes, and various anti-IgA antibodies can be used. For example, antibodies that specifically recognize abnormal glycosylated IgA, such as galactose-deficient IgA, can also be used as anti-IgA antibodies in the invention, and can be used to recover IgA immune complexes.

The method for measuring the IgA nephropathy marker protein in the recovered IgA immune complexes is not particularly limited, as long as it is a method that can generally measure the concentration of the target protein. For example, the IgA nephropathy marker protein can be measured by an immunological method using an antibody against the IgA nephropathy marker protein. Examples of such immunological methods include ELISA (enzyme-linked immunosorbent assay), latex agglutination, and immunochromatography. These methods can be performed by conventional methods using an antibody against the IgA nephropathy marker protein. The IgA nephropathy marker protein can also be measured by a method using mass spectrometry, such as liquid chromatography-mass spectrometry, or the like.

The "amount of IgA nephropathy marker protein in IgA immune complexes" may be the value obtained by quantifying the IgA nephropathy marker protein itself, but is preferably a "relative value to the amount of IgA protein in IgA immune complexes", since this allows for more accurate comparison of the amounts of IgA nephropathy marker protein between samples. Specifically, step (a) is preferably carried out in the following steps (c-1) to (c-5). Steps (c-1) to (c-3) can be carried out in the same manner as described above.

(c-1) separating serum or plasma from a blood sample collected from a subjected animal;
(c-2) recovering IgA immune complexes contained in the serum or plasma obtained in step (c-1);
(c-3) measuring the IgA nephropathy marker protein in the IgA immune complex recovered in step (c-2);
(c-4) measuring the IgA protein in the IgA immune complexes recovered in step (c-2);
(c-5) dividing the amount of IgA nephropathy marker protein measured in step (c-3) by the amount of IgA protein measured in step (c-4) ([amount of IgA nephropathy marker protein in IgA immune complexes]/[amount of IgA protein in IgA immune complexes]) to determine the amount of IgA nephropathy marker protein contained in the IgA immune complexes in the blood sample collected from the subjected animal.

The measurement of IgA protein in step (c-4) can be carried out, for example, by an immunological technique using an anti-IgA antibody or a method utilizing mass spectrometry such as liquid chromatography mass spectrometry. Examples of such immunological techniques include ELISA, latex agglutination, immunochromatography, or the like. In step (c-5), since the "amount of IgA nephropathy marker protein per amount of IgA protein" ([amount of IgA nephropathy marker protein in IgA immune complex]/[amount of IgA protein in IgA immune complex]) is easily measured, it is preferable to measure the amount of IgA protein in step (c-4) by the same method as that used to measure the amount of IgA nephropathy marker protein in step (c-3).

In the method for determining IgA nephropathy according to the invention, in step (b), the presence or absence of IgA nephropathy, or the likelihood of onset of IgA nephropathy in a subjected animal, is determined based on the amount of the IgA nephropathy marker protein measured in step (a). The amount of the IgA nephropathy marker protein used in the present invention contained in IgA immune complexes in the blood tends to be significantly higher in patients with IgA nephropathy than in the healthy subject group. Therefore, the amount of the IgA nephropathy marker protein contained in IgA immune complexes in the blood of the subjected animal can be used to determine the presence or likelihood of onset of IgA nephropathy in the subjected animal.

A threshold value for distinguishing between IgA nephropathy patients and nonpatients is set in advance, the amount of IgA nephropathy marker protein contained in IgA immune complexes in the blood of a subjected animal is measured, and it is determined whether the amount of the protein is equal to or greater than the predetermined threshold value for distinguishing between the IgA nephropathy patient group and the healthy subject group. If the measured amount of IgA nephropathy marker protein is equal to or greater than the predetermined threshold value, it is determined that the subjected animal has a high likelihood of onset of (affected) IgA nephropathy.

The threshold value for distinguishing the IgA nephropathy patient group from the healthy subject group can be experimentally determined, taking into consideration the method for measuring the IgA nephropathy marker protein, etc. For example, the threshold value can be appropriately determined by performing the method for determining IgA nephropathy according to the present invention on blood collected from a group known not to have IgA nephropathy and on blood collected from a group known to have IgA nephropathy, measuring the amount of IgA nephropathy marker protein contained in IgA immune complexes in each blood sample, and comparing the measured values for both groups.

As shown in the Examples below, in patients with IgA nephropathy, the amount of IgA nephropathy marker protein contained in IgA immune complexes in the blood tends to decrease rapidly after aggressive treatment. Therefore, it is expected that the amount of IgA nephropathy marker protein contained in IgA immune complexes in the blood is positively correlated with the disease activity of IgA nephropathy. Therefore, by setting an appropriate threshold value, it is possible to accurately assess not only the presence or absence of IgA nephropathy or the likelihood of onset of IgA nephropathy, but also the disease activity of IgA nephropathy, and the obtained assessment is preferably provided to physicians as information useful for formulating a treatment plan. That is, the method for determining IgA nephropathy according to the present invention can also determine the disease activity of IgA nephropathy. The higher the amount of IgA nephropathy marker protein measured in step (a), the higher the disease activity is assessed to be.

Based on the expectation that there is a positive correlation between the amount of IgA nephropathy marker protein contained in IgA immune complexes in blood and the disease activity of IgA nephropathy, the disease activity of IgA nephropathy in a subjected animal can also be assessed by measuring the amount of IgA nephropathy marker protein contained in IgA immune complexes in blood samples collected from the same subjected animal over time and comparing these amounts. For example, for an animal diagnosed with IgA nephropathy, if the amount of IgA nephropathy marker protein measured in step (a) is lower than the amount of IgA nephropathy marker protein contained in IgA immune complexes in blood samples collected from the same subjected animal before the blood sample used for measurement was collected, it can be determined that the disease activity of IgA nephropathy in the subjected animal has decreased and the pathological condition has improved. The obtained assessment is preferably provided to a physician as information useful for formulating a treatment plan.

In the method for determining IgA nephropathy according to the invention, the subjected animal assessed as having a high likelihood of onset of IgA nephropathy and the subjected animal assessed as having a high disease activity have high amounts of IgA nephropathy marker protein contained in IgA immune complexes in their blood. Therefore, for the subjected animal determined as having a high likelihood of onset or high disease activity of IgA nephropathy in the method for determining IgA nephropathy according to the invention, receiving aggressive treatments such as corticosteroid therapy, tonsillectomy, or the like is predicted to be effective. Thus, the method for determining IgA nephropathy according to the present invention can be performed on the patients with IgA nephropathy who have been diagnosed with IgA nephropathy to obtain information for selecting a treatment method.

Since FHRP1 is one of the regulatory factors of complement pathways such as the alternative pathway or the lectin pathway, if the amount of IgA nephropathy marker protein contained in the IgA immune complexes in the blood is high, the IgA nephropathy that develops in the subjected animal is likely to be caused by complement activation. Therefore, in the method for determining IgA nephropathy of the present invention, it is predicted that treatment with a complement inhibitor targeting the alternative pathway or the lectin pathway will be effective for the subjected animals assessed as having a high likelihood of onset or high disease activity of IgA nephropathy. In other words, the method for determining IgA nephropathy of the present invention can provide useful information for selecting patients who should receive complement therapeutic agents and predicting the effectiveness of treatment. Examples of the complement therapeutic agents for the alternative pathway include LNP023 (manufactured by Novartis), and examples of the complement therapeutic agents for the lectin pathway include Narsoprimab (manufactured by Omeros).

The method for determining IgA nephropathy according to the invention can be carried out more easily by preparing a kit containing reagents, devices, etc. used for recovering IgA immune complexes and quantifying IgA nephropathy marker proteins and IgA proteins. The kit for determining the likelihood of onset or disease activity of IgA nephropathy in a subjected animal preferably contains an anti-IgA antibody and an antibody against the IgA nephropathy marker protein.

The kit for determining the disease activity of IgA nephropathy in a subjected animal according to the present invention can be used to predict the effectiveness of treatment for a patient with IgA nephropathy.

In one embodiment, the invention provides use of an anti-IgA antibody and an antibody against an IgA nephropathy marker protein for producing a kit for predicting the effectiveness of treatment for a patient with IgA nephropathy.

In one embodiment, the invention provides an anti-IgA antibody and an antibody against an IgA nephropathy marker protein for predicting the effectiveness of treatment for a patient with IgA nephropathy.

In one embodiment, the present invention may include other steps in addition to steps (a) and (b). Examples of other steps include (c) treating a patient with IgA nephropathy based on the assessment results of the likelihood of onset or disease activity of IgA nephropathy determined in step (b). When step (c) is further included in addition to steps (a) and (b), the method of the present embodiment may be a method for treating a patient with IgA nephropathy.

In step (c), a treatment policy for IgA nephropathy can be determined based on the assessment results of the determined likelihood of onset or disease activity of IgA nephropathy. Treatment for IgA nephropathy patients assessed as having a high likelihood of onset or high disease activity of IgA nephropathy may be a treatment with a complement inhibitor targeting the alternative pathway or the lectin pathway, or aggressive treatment such as corticosteroid treatment and tonsillectomy. Among these, either or both of complement inhibitor treatment targeting the alternative pathway or the lectin pathway and corticosteroid treatment are preferable.

The present invention may include the following aspects.

A method for treating a patient with IgA nephropathy, comprising:
(a) measuring an IgA nephropathy marker protein contained in an IgA immune complex in a blood sample collected from a subjected animal;
(b) determining a likelihood of onset or disease activity of IgA nephropathy in the subjected animal based on an amount of the IgA nephropathy marker protein measured in step (a);
(c) treating a patient with IgA nephropathy based on the assessment result of the likelihood of onset or disease activity of IgA nephropathy determined in step (b);
wherein
the IgA nephropathy marker protein is a protein contained in an IgA immune complex, and a protein that is contained in a greater amount in an IgA immune complex of an IgA nephropathy patient group than in an IgA immune complex of a healthy subject group.

### Examples

The present invention will now be described in more detail with reference to the Examples, but the invention is not limited to the following Examples.

### [Example 1]

To identify novel biomarker candidate proteins that can diagnose IgA nephropathy and determine disease activity by measuring blood levels without performing kidney biopsies in patients with IgA nephropathy, proteins that are specifically increased in patients with IgA nephropathy were searched by performing proteomic analysis of IgA immune complexes in the blood.

### (1) Collection of serum samples

Sera were collected from healthy subjects (n = 20), a group of patients with other renal diseases (other than IgA nephropathy) such as membranous nephropathy and minimal change nephrotic syndrome (n = 8), a supportive treatment group of IgA nephropathy patients (n = 8), and an aggressive treatment group of IgA nephropathy patients (n = 24) (Table 1). Patients with IgA nephropathy were classified into a supportive treatment group and an aggressive treatment group based on the treatment algorithm of Fujita Health University.

The supportive treatment group includes patients with advanced renal dysfunction, patients with minimal acute lesions in renal biopsy tissue, or patients with mild urinalysis findings. Conservative treatment centered on RA system inhibitors was used. On the other hand, the aggressive treatment group includes patients treated with tonsillectomy and steroid pulse therapy (Pozzi et al.'s method). Patients with histologically confirmed acute lesions and severe urinalysis findings fall into this group.

Blood samples were collected from patients, not from healthy subjects, at the time of kidney biopsy. For patients with other renal diseases (renal diseases other than IgA nephropathy) and some patients with IgA nephropathy, blood samples were also collected approximately 2 years after the start of treatment. The details of clinical samples and their clinical information are shown in Tables 1 and 2. Clinical information for the IgA nephropathy group and the other renal disease group before and after treatment is shown in Table 3.

**[Table 1]**

| | | |
|---|---|---|
| Healthy subject group | 20 | |
| Renal disease (other than IgA nephropathy) group | 8 | In 8 cases, samples were collected approximately 2 years after the start of treatment. |
| IgA nephropathy patients Supportive treatment group | 8 | In 8 cases, samples were collected approximately 2 years after the start of treatment. |
| IgA nephropathy patients Aggressive treatment group | 24 | In 10 cases, samples were collected approximately 2 years after the start of treatment. |

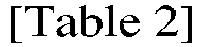

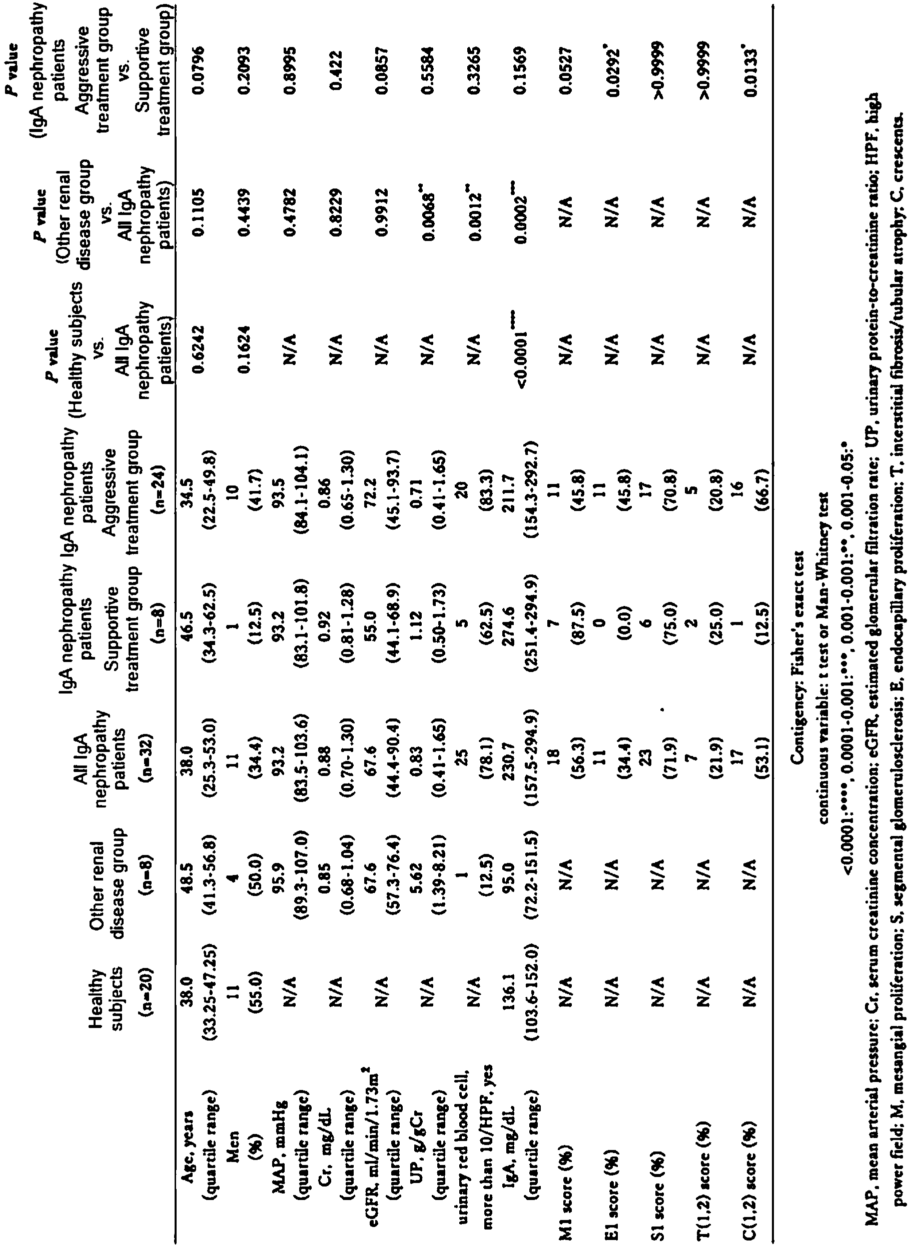

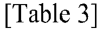

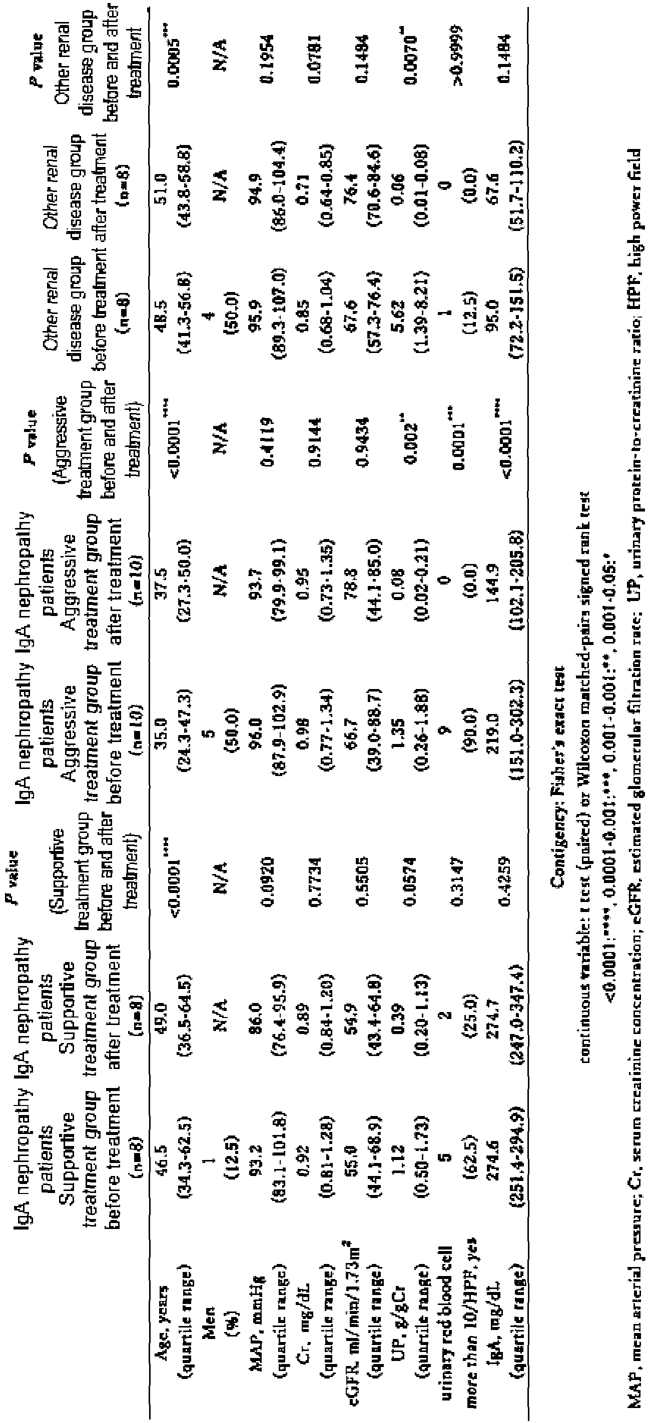

### (2) Separation and purification of IgA immune complex from serum

IgA immune complexes were isolated and purified from 100 µL of each serum sample by affinity chromatography using a cyanogen bromide-activated Sepharose column coupled with anti-human IgA antibody (product number: 0855068, manufactured by Cappel Laboratories). Specifically, 100 µL of serum was mixed with 1 mL of phosphate-buffered saline, filtered through a 0.45 µm filter, and then adsorbed onto the affinity column. After washing the affinity column with phosphate-buffered saline, the IgA immune complexes were eluted with 0.1 M glycine-HCl buffer (pH 2.7). The eluted solution was neutralized with 1.0 M Tris-HCl buffer (pH 9.0).

The separated and purified IgA immune complexes were concentrated by centrifugation at 2330 × g and 4°C for 20 minutes using an ultrafiltration membrane (product name: Ultra-4 30K Centrifugal Filter Units, manufactured by Amicon). The absorbance at a wavelength of 280 nm was then measured using a Nano Vue spectrophotometer (manufactured by GE), and the protein concentration was calculated.

### (3) Proteome analysis of IgA immune complexes

5 µg of IgA immune complexes were precipitated using chilled acetone. The precipitate was dissolved in 250 mM sodium phosphate buffer (pH 6.0), and 2.5 mU of sialidase A (product name: GK80040, manufactured by Prozyme) was added. The mixture was then subjected to an overnight enzyme treatment at 37°C. Dithiothreitol (DTT) was then added to a final concentration of 20 mM, and the mixture was reduced at room temperature for 15 minutes. For the reduced sample, 0.1 g of trypsin (product name: V528A, manufactured by Promega) dissolved in 100 mM ammonium bicarbonate solution (pH 8.3) was added, and the mixture was again subjected to an overnight enzyme treatment at 37°C. The entire sample after the enzyme treatment was desalted using a C18 spin column (product name: 89870, manufactured by Thermo Fisher Scientific). Finally, the sample was dried using a SpeedVac.

The dried sample was dissolved in 20 µL of 0.1% formic acid to a protein concentration of 0.1 µg/L and a final DTT concentration of 20 mM. The sample was then analyzed using liquid chromatography-high-resolution mass spectrometry (LC-HRMS), and a label-free quantification method was employed for a comprehensive analysis. Mass spectrometry was performed using an EASY-nLC 1000 system and an Orbitrap Fusion mass spectrometer (both manufactured by Thermo Fisher Scientific). Liquid chromatography was performed using a gradient of 0% to 35% acetonitrile at a flow rate of 300 nL/min over 120 minutes.

Based on the mass spectral information obtained above, a database search [MASCOT (version 2.6.1, Matrix Science), EQUEST HT] was performed using "Proteome Discoverer 2.2 software" (manufactured by Thermo Fischer Scientific) to identify proteins and perform label-free quantification.

### (4) Protein differential analysis using MS (mass spectrometry) data

The relative abundance of proteins identified by Proteome Discoverer was analyzed using Perseus (version 1.6.15.0). Protein relative abundance was Log2 transformed and normalized by subtracting the median value for each sample. Missing values were imputed based on a normal distribution (width = 0.3, downshift = 1.8). Statistical significance was determined using Student's t-test, and proteins with a P-value < 0.05 and an absolute Log2 (fold change) value > 0.58 were considered to be significantly different proteins.

To comprehensively quantitatively analyze proteins in IgA immune complexes that change depending on the pathology of IgA nephropathy, the relative abundance of proteins identified by mass spectrometry was compared before and after treatment in IgA nephropathy patients, healthy subjects, and IgA nephropathy patients (aggressive treatment group).

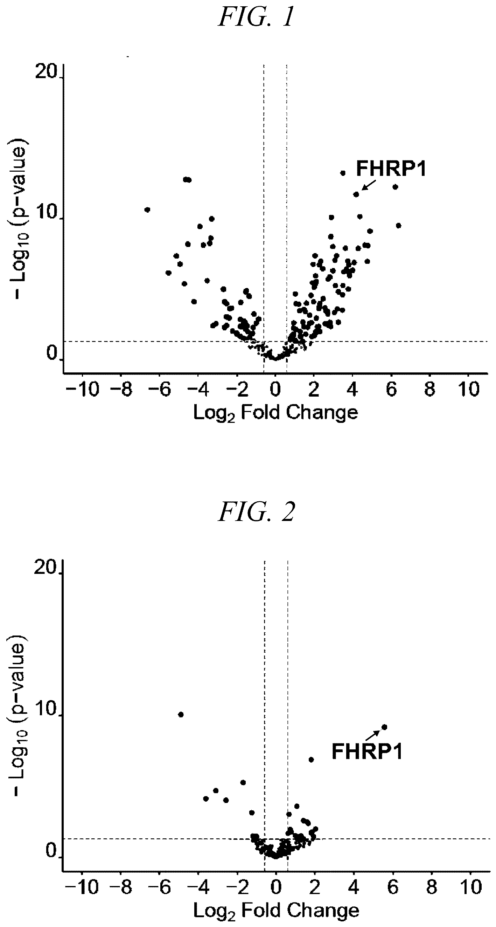

FIG. 1 shows the results of comparing the relative abundance of proteins in IgA immune complexes between the IgA nephropathy group (supportive treatment group + aggressive treatment group) (at the time of renal biopsy) and the healthy subject group. In FIG. 1, the horizontal axis represents the logarithm of the relative ratio of protein abundance between the IgA nephropathy group and the healthy subject group [Log2 (Fold change)], and the vertical axis represents the P-value [-Log10 (P-value)]. Black dots shifted in the positive direction of the logarithm [Log2 (Fold change)] indicate proteins that were abundant in IgA nephropathy, and black dots shifted in the negative direction indicate proteins that were abundant in healthy subjects. In IgA nephropathy patients, FHRP1 was present in higher amounts in IgA immune complexes than in healthy subjects. Proteins, including FHRP1, that were significantly more abundant in IgA nephropathy patients are shown in Table 4.

**[Table 4]**

| Description | Accession | -Log (P-value) | Difference |
|---|---|---|---|
| Alpha-1-antitrypsin | P01009 | 13.24479898 | 3.500573048 |
| Apolipoprotein C-I | P02654 | 12.24891464 | 6.210885555 |
| Complement factor H-related protein 1 | Q03591 | 11.71999225 | 4.179673799 |
| Cathepsin G | P08311 | 9.10697991 | 4.896997154 |
| Solute carrier family 13 member 4 | Q9UKG4 | 8.13048304 | 4.632756284 |
| Alpha-2-macroglobulin | P01023 | 8.02901637 | 2.975041415 |
| Myeloperoxidase | P05164 | 6.96655578 | 4.762692290 |
| Complement C1q subcomponent subunit B | P02746 | 6.42151850 | 3.677924318 |
| Rho GTPase-activating protein 42 | A6NI28 | 6.34094089 | 3.986908359 |
| Complement C1q subcomponent subunit C | P02747 | 5.25843577 | 3.499435689 |

FIG. 2 shows the comparison of proteins in IgA immune complexes before and after treatment in the aggressively treated IgA nephropathy group. In FIG. 2, the horizontal axis represents the logarithm of the relative ratio of protein abundance before/after treatment (approximately 2 years later) in the aggressively treated IgA nephropathy group [Log2 (Fold change)], and the vertical axis represents the P-value [-Log10 (P-value)]. Black dots shifted in the logarithm [Log2 (Fold change)] in the positive direction indicate proteins abundant before treatment, while black dots shifted in the negative direction indicate proteins abundant after treatment. Aggressive treatment of IgA nephropathy was found to decrease FHRP1 in IgA immune complexes. Thus, FHRP1 was found to be significantly more abundant in IgA nephropathy patients than in healthy subjects and to be significantly decreased by aggressive treatment (FIG 1 and 2).

### (5) Analysis of IgA immune complexes by ELISA

Using ELISA, an immunological technique, the amount of FHRP1 contained in IgA immune complexes was measured in healthy subjects, patients with IgA nephropathy, and patients with other renal diseases (n = 7 due to insufficient samples). FHRP1 was measured by measuring the IgA concentration, and the amount of FHRP1 contained per 10 µg of IgA (ng/10 µg of IgA) was calculated. The measurement values were analyzed using the Kruskal Wallis test (P < 0.0001), followed by Dunn's multiple comparisons test.

Specifically, the IgA concentration of the IgA immune complex solution collected in (2) above was calculated using a commercially available ELISA kit for IgA measurement (product name: "IgA Human Uncoated ELISA kit," manufactured by Thermo Fischer Scientific). Using the measurement value, the IgA concentration was adjusted to 10 µg/mL, and the FHRP1 concentration was measured using a commercially available ELISA kit for human CFHR measurement (product name: "RayBio (registered trademark) Human CFHR1 ELISA kit", manufactured by RayBiotech) according to the instructions attached to the kit.

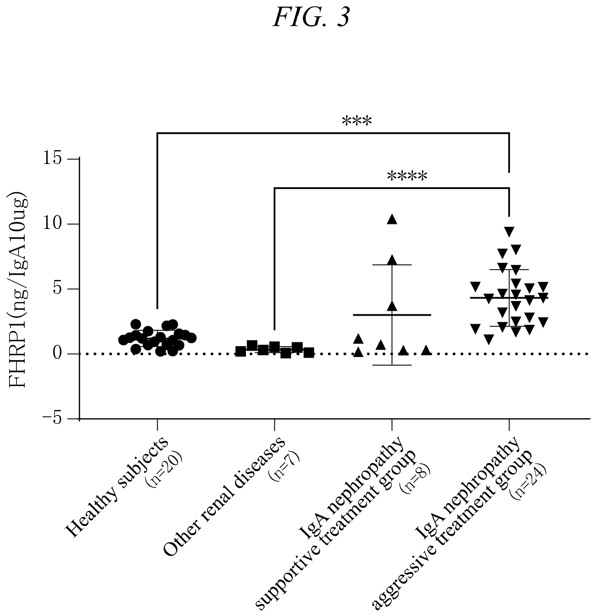

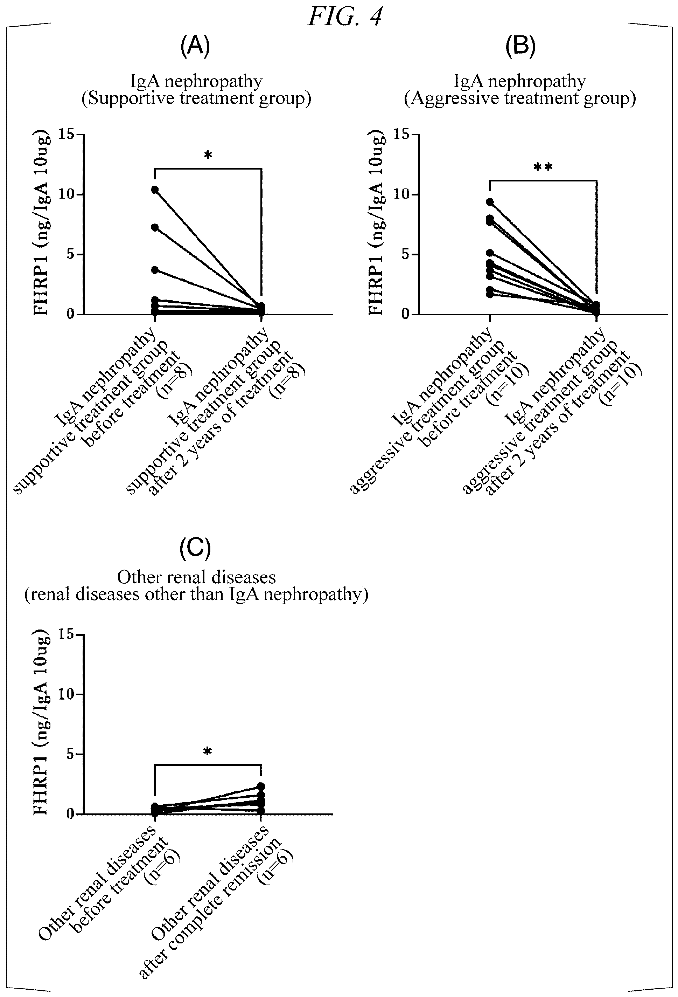

The measurement results of the amount of FHRP1 contained in the IgA immune complexes (amount of FHRP1 contained per 10 µg of IgA [ng/10 µg of IgA]) in each group are shown in FIG. 3. The results of comparing the FHRP1 levels before and after the treatment in each patient group are shown in FIG. 4.

As shown in FIG. 3, a significant difference was observed between the healthy subjects and the aggressive treated IgA nephropathy group (P = 0.0001). A significant difference was also observed between the aggressively treated IgA nephropathy group and the other renal disease group (P < 0.0001). Thus, the amount of FHRP1 contained in IgA immune complexes was significantly higher in the IgA nephropathy patients than in the healthy subjects and the patients with other renal diseases (one-way ANOVA, Tukey's multiple comparison test, P < 0.0001, P = 0.0002), and the aggressively treated group, which is the most severe group of IgA nephropathy, showed high levels. No significant differences were observed between the patients with other renal diseases and the healthy subjects, demonstrating that FHRP1 in IgA immune complexes is specifically elevated in the IgA nephropathy patients. As shown in FIG. 4, the level of FHRP1 binding to IgA significantly decreased after treatment compared to before treatment in both the IgA nephropathy supportive treatment group (FIG. 4 (A)) and the aggressive treatment group (FIG. 4 (B)) (Wilcoxon test, P = 0.0156, 0.0020). In the group of patients with other renal diseases (FIG. 4 (C)), the level significantly increased after treatment compared to before treatment (Paired t test, P = 0.0471).

While preferable embodiments of the invention have been described and illustrated, it should be understood that these are exemplary of the present invention and should not be considered limiting. Additions, omissions, substitutions, and other modifications can be made without departing from the gist or scope of the present invention. Accordingly, the present invention is not to be deemed limited by the foregoing description, but is limited only by the appended claims.

## Claims

1. A method for determining IgA nephropathy, which determines a likelihood of onset or disease activity of IgA nephropathy in a subjected animal, comprising:
(a) measuring an IgA nephropathy marker protein contained in an IgA immune complex in a blood sample collected from a subjected animal; and
(b) determining a likelihood of onset or disease activity of IgA nephropathy in the subjected animal based on an amount of the IgA nephropathy marker protein measured in step (a), wherein
the IgA nephropathy marker protein is a protein contained in an IgA immune complex, and a protein that is contained in a greater amount in an IgA immune complex of an IgA nephropathy patient group than in an IgA immune complex of a healthy subject group.

2. The method for determining IgA nephropathy according to claim 1, wherein
step (a) is carried out by:
(c-1) separating serum or plasma from the blood sample collected from the subjected animal;
(c-2) recovering the IgA immune complex contained in the serum or the plasma obtained in step (c-1); and
(c-3) measuring the IgA nephropathy marker protein in the IgA immune complex recovered in step (c-2).

3. The method for determining IgA nephropathy according to claim 2, wherein
in step (c-2), the recovery of the IgA immune complex is carried out by affinity chromatography using an anti-IgA antibody.

4. The method for determining IgA nephropathy according to any one of claims 1 to 3, wherein
the IgA nephropathy marker protein in the IgA immune complex is measured by an immunological technique using an antibody against the IgA nephropathy marker protein.

5. The method for determining IgA nephropathy according to any one of claims 1 to 3, wherein
the IgA nephropathy marker protein is one or more selected from the group consisting of alpha 1-antitrypsin, apolipoprotein C-1, FHRP1, cathepsin G, SLC13A4, alpha 2-macroglobulin, myeloperoxidase, C1QB, RhoGAP42, and C1QC.

6. The method for determining IgA nephropathy according to any one of claims 1 to 3, wherein
in step (b), if the amount of the IgA nephropathy marker protein measured in step (a) is greater than a predetermined threshold value for distinguishing between an IgA nephropathy patient group and a healthy subject group, the subjected animal is determined to have a high likelihood of onset of IgA nephropathy.

7. The method for determining IgA nephropathy according to claim 6, wherein
if the subjected animal is determined to have a high likelihood of onset of IgA nephropathy, it is predicted that corticosteroid treatment will be effective for the subjected animal.

8. The method for determining IgA nephropathy according to claim 6, wherein
if the subjected animal is determined to have a high likelihood of onset of IgA nephropathy, it is predicted that treatment with a complement inhibitor targeting an alternative pathway or a lectin pathway will be effective for the subjected animal.

9. The method for determining IgA nephropathy according to any one of claims 1 to 3, wherein
in step (b), if the amount of the IgA nephropathy marker protein measured in step (a) is lower than the amount of the IgA nephropathy marker protein contained in the IgA immune complex in the blood sample collected from the subjected animal before a time the blood sample provided in step (a) was collected, it is determined that the disease activity of IgA nephropathy in the subjected animal has decreased.

10. A kit for determining disease activity of IgA nephropathy in a subjected animal, comprising:
an anti-IgA antibody; and
an antibody against an IgA nephropathy marker protein, wherein
the IgA nephropathy marker protein is a protein contained in an IgA immune complex, and a protein that is contained in a greater amount in an IgA immune complex of an IgA nephropathy patient group than in an IgA immune complex of a healthy subject group.

11. The kit for determining IgA nephropathy according to claim 10, wherein
the IgA nephropathy marker protein is one or more selected from the group consisting of alpha 1-antitrypsin, apolipoprotein C-1, FHRP1, cathepsin G, SLC13A4, alpha 2-macroglobulin, myeloperoxidase, C1QB, RhoGAP42, and C1QC.
